# EUROPEAN PATENT SPECIFICATION

(11) **EP 1 759 716 B1**
(45) Date of publication and mention of the grant of the patent: **26.11.2008**
(21) Application number: 06380208.6
(22) Date of filing: 21.07.2006
(51) Int. Cl.: A61L 9/01

(54) **Composition for neutralising unpleasant odours**
Stoffgemisch zur Beseitigung unangenehmer Gerüche
Composition pour neutraliser des odeurs désagréables

(30) Priority: 29.08.2005 ES 200502116
(43) Date of publication of application: 07.03.2007
(73) Proprietor: Sensient Fragrances S.A., Armilla, 18100 Granada (ES)
(72) Inventor: Ruiz Sanchez, Antonio, 18100 Armilla Granada (ES)
(74) Representative: Carpintero Lopez, Francisco

(56) References cited:
- WO-A-98/56337
- WO-A2-01/16266
- US-A1- 2003 199 402

## Description

### FIELD OF THE INVENTION

The present invention relates to the field of air freshening, in particular to a composition for neutralizing unpleasant environmental odours, based on a triple system of chemical, physical and biological neutralization of unpleasant odours.

### BACKGROUND OF THE INVENTION

Human activity generates a great many unpleasant odours in the environment. The nature of these unpleasant odours is highly varied both on account of the physical state of the unpleasant odour particles and their chemical characteristics or their origin (biological decomposition, chemical agents, smoking, etc.). In addition, these unpleasant odours are generated in spaces or environments of everyday use such as the bathroom, kitchen, refuse, closed environments with fumes (bars), etc. In this respect, many systems have been developed to combat such unpleasant odours.

We are familiar with deodorizing compositions based on chemical systems for neutralizing unpleasant odours. Zinc ricinoleate, for instance, is a compound that may be bonded chemically to strong-smelling organic substances containing chemical groups with sulphur or nitrogen, such as for example mercaptanes, thioethers, low molecular weight carboxylic acids or amines, thus neutralizing or reducing their unpleasant smell. This means that zinc ricinoleate has a number of applications in cleaners, air fresheners, deodorants and in other domestic applications. Patents EP1250938, US 2003/0133892 or CN 1113809 describe compositions comprising zinc ricinoleate for different applications.

We also know that certain compounds are suitable for use as bactericides or microbicides, thereby preventing the appearance of unpleasant odours caused by the activity of microorganisms, as well as possible infections caused by them. In this respect, essential oils and/or their components have already been used in the field of air freshening for this microbicidal purpose.

US2003/199402 discloses a composition for reducing malodours comprising zinc ricinoleate, saturated aliphatic esters, polyvinyl pyrrolidone, essential oil, aromatic compound. The composition may be used for air freshening by supplying it to the air, e.g. by spraying.

WO 01/16266 discloses a composition for reducing malodours comprising zinc ricinoleate, saturated aliphatic esters, polyvinyl pyrrolidone, essential oil, aromatic compound. The composition may be used for air freshening by supplying to air, e.g. by spraying.

An example of this microbicidal use of essential oils and/or their components is described in patent application W02004/035097.

The present invention according to claim 1 addresses the need for an active odour control system which involves, besides the combination of at least one chemical odour neutralizing system and at least one biological neutralizing system, a physical odour neutralizing system, based on the one hand on the capacity of certain compounds to modify the vapour pressure of the gaseous particles of the unpleasant odour and, on the other, on the capacity of other compounds to capture and precipitate the solid particles in suspension permitting their elimination in the normal cleaning process.

### OBJECT OF THE INVENTION

First of all, the object of the invention is a composition for neutralizing unpleasant odours in the environment according to claim 1.

By means of the chemical neutralization system the composition of the invention acts chemically on the molecules of the unpleasant odour generated by human activity (cooking, smoking, bathrooms...) so that odourless particles are obtained.

As explained previously, the physical neutralization system is based on modifying the vapour pressure of the gaseous particles of the unpleasant odour, as well as on the precipitation of the solid particles in suspension in the environment, as may be the case of pollen, bacteria, dust, etc., thereby eradicating the focal points of dirt and unpleasant odours.

Finally, the biological neutralization system permits the composition of the present invention to prevent the appearance of the unpleasant odours due to bacterial decomposition thanks to the antibacterial and/or bacteriostatic characteristics of essential oils and/or their components, as well as to act as an environmental disinfectant.

The combination of these odour neutralizing systems together optionally with any suitable fragrance make the composition of the present invention not only an appropriate means for eradicating the unpleasant odours that we perceive, but also an unparalleled control and protection system against internal or external agents that may produce unpleasant odours.

Another object of the present invention refers to the use of the composition for neutralizing the unpleasant odours in the environment according to claim 1 by means of their dispersion in the environment.

### DETAILED DESCRIPTION OF THE INVENTION

The main aspect of the invention refers to a composition for neutralizing unpleasant odours in the environment according to claim 1.

The purpose of a composition like that described here is to provide a system for the active control of odours thanks to a triple physical, chemical and biological system, so that not only are the molecules of the unpleasant odour neutralized directly, but also the causes that propitiate the appearance of these unpleasant odours. The composition of the invention enables us to obtain clean, aromatized and microorganism-free environments, thereby also preventing the outbreak of infections.

In the context of the present application chemical neutralization system means any system formed of zinc salts, and preferably zinc ricinoleate, a molecule capable of binding chemically to strong-smelling organic substances containing chemical groups with sulphur or nitrogen, thereby reducing or neutralizing its unpleasant odour.

By physical neutralization system we mean all those molecules or compounds that modify the intrinsic physical properties of molecules of unpleasant odour or act on those particles that propitiate the appearance of unpleasant odours. Due to the fact that the physical state of said unpleasant-odour-generating molecules or particles may be gaseous or else they may be solid or even liquid particles in suspension, it is necessary for the physical neutralization system to act globally on all such particles.

Therefore, the physical neutralization system comprises, first of all, compounds capable of modifying the vapour pressure of the gaseous molecules of the unpleasant odour. When the vapour pressure of the molecules decreases, our sense of smell is not capable of perceiving them, so it is a case therefore of pseudoelimination. In one particular embodiment of the invention, the compounds capable of modifying the vapour pressure of the particles of unpleasant odour are saturated aliphatic esters (C10-C20) or methyl esters of resinic acids, preferably isopropyl myristate and hercolyn, respectively. The physical neutralization of the invention also comprises a coadjuvant which helps to bind and precipitate the solid particles suspended in the environment (bacteria, pollen, dust, etc.) making them fall to the ground so that they may be removed in the normal cleaning process with a broom, mop, duster, etc. This coadjuvant is extremely important in the context of the present invention as the solid particles in suspension usually form nuclei that generate dirt and therefore unpleasant odours. The appropriate compounds to act as coadjuvants in the present invention are polyvinyl pyrrolidone (PVP) or derivatives of same. As a PVP derivative applicable for the purposes of the invention we may mention the copolymer of vinyl pyrrolidone and dimethylaminopropyl methacrylamide.

The function of the biological neutralization system of the composition of the invention is to kill or inhibit the growth of microorganisms that may potentially generate unpleasant odours in the air as well as on surfaces. The biological neutralization system also provides environmental disinfection which assists the non-appearance of infections caused by possible microorganisms present in the environment. In the context of the composition of the invention the microbicidal effect is obtained thanks to the presence of essential oils and/or of their active ingredients. These essential oils may be obtained from the tea tree, Ceylon cinnamon leaves, citronella, artemisia, lemongrass, thyme, citrus fruits, anise, geranium, cedar, clove, rose, mint, lavender, eucalyptus, spearmint, sandalwood, pine, bergamot, lavandine or mixtures thereof; and the active ingredients are selected from amongst thymol, citronellal, citronellol, estragol, geranyl acetate, eucalyptol, geraniol, menthol, cedrol, anethol, limonene, acetol, carbacrol, eucalyptol, eugenol, terpineol, cynamaldehyde, cynamic acid, citral, nerol, dihydromircenol, rose oxide, limonene and mixtures thereof.

Besides the afore-mentioned systems for neutralizing unpleasant odours, the composition in accordance with the invention may also contain one or more aromatic compounds whose sole function is to provide a more agreeable smell in line with the composition. The aromatic compounds used in the present invention encompass but are not confined to such chemical compounds as aldehydes (phenyl acetic, C8, C9, C10, etc.), acetates (benzyl, hexyl, octyl, etc) ionones (gamma-methyl, beta, etc.), lactones (gamma undecalactone, decalactone, nonalactone, dodecalactone, etc.) alcohols (phenyl ethylic, phenylpropylic), pyrazines or acids.

In another aspect, the invention makes reference to the use of a composition, as that described above, for the eradication of unpleasant odours which comprises their dispersion in the environment by any conventional means of dispersion. The conventional means of dispersion comprise but are not confined to electric air fresheners, sprays, automobile air fresheners, wick air fresheners, gelatine air fresheners, candles, etc.

The following examples serve to illustrate the composition of the invention as well as the effect of chemical, physical and biological neutralization that it possesses.

### Example1: Composition for neutralizing unpleasant odours

One particular embodiment of the invention is obtained by means of the preparation of a formulation in accordance with table 1 in which the percentages are specified by weight:

**Table 1**

| **COMPONENTS** | **BY WEIGHT %** |
|---|---|
| Elemi | 1 |
| Anise | 1 |
| Eucalyptus | 1.5 |
| Zinc ricinoleate | 12 |
| Petigraim | 0.8 |
| Isopropyl Myristate | 10 |
| Basilicon | 0.6 |
| Sage | 0.2 |
| Polyvinyl Pyrrolidone | 10 |
| Orange | 3 |
| Bergamot | 5 |
| Limetta | 4 |
| Cinnamon | 0.2 |
| Clove | 0.5 |
| Cedar | 8 |
| Ethanol | 26,8 |
| Geranium | 12 |
| Litsea Cubeba | 0.8 |
| Mint | 0.2 |
| Thymol | 0.4 |
| **TOTAL** | **100** |

### Example 2: Chemical neutralization

The chemical neutralization analyses were carried out in 1.3 m³ booths. The contaminants were evaporated and then the booth was sprayed with an air freshener with a composition in accordance with the present application. The booth had a ventilation system to speed up evaporation.
The working procedure followed consisted first of all in spraying the 1.3 m³ booth with a mixture of gases that is the object of study composed of:

After 10 minutes,

| |
|---|
| Dimethyl Sulphide |
| Furfural/Pyroligneous acid |
| Isovalerianic acid |
| Caproic acid |
| p-Cresol |
| Nicotine |

once the mixture of contaminants is homogeneous, the first sample was collected by means of solid phase microextraction (SPME) technology. The contaminants extracted were analyzed by gas chromatography/mass spectrometry (GC/MS). Once the control sample was collected, the composition that is the object of the invention was set into operation with a cold air ventilation system for an hour. At the end of that period another sample was collected by means of SPME and analyzed again by GC/MS. The experiment was repeated in triplicate and the mean values of all the experiments were calculated statistically.
These results showed that the reduction in the quantity of contaminants in the environment was 54.9%.

### Example 3: Physical neutralization

The analysis of physical neutralization was done by measuring the dusttrapping effect of the solution proposed by the invention. For this purpose a dusty mixture was dispersed in a chamber similar to that of the previous example. The composition in accordance with the invention was then sprayed for 3 seconds. After 10 minutes a gravimetric analysis or reading was taken at a height of 1.5 metres inside the actual booth. The results showed that the presence of dust in the environment was reduced by around 26%.

### Example 4: Biological neutralization

The neutralization test consisted of mixing 1 ml of the composition of the invention with suspension cultures of Pseudomona aeruginosa and Enterococcus hirae obtained by means of sowing in test tubes with 10⁹ ufc/ml and grown for 16 hours at 20°C. After 5 minutes' contact between the cultures and the test composition, the solution was sown on a petri dish and incubated for a period of 24h-48h. The results showed that a reduction in bacterial load of between 95%-100% took place both for Pseudomona aeruginosa and for Enterococcus hirae.

## Claims

1. Composition for neutralizing unpleasant odours in the environment which comprises
- at least a chemical neutralization system based on a zinc salt;
- at least a physical neutralization system further comprising a compound capable of lowering the vapour pressure of the gaseous molecules of the unpleasant odour and a coadjuvant capable of precipitating the solid particles in suspension in the air;
- and at least a biological neutralization system based in at least one essential oil or its active ingredient,
**characterised in that** the coadjuvant is based on a copolymer of vinylpyrrolidone and dimethyl aminopropyl methacrylamide.

2. Composition for neutralizing unpleasant odours in the environment in accordance with claim 1 **characterized in that** said zinc salt is zinc ricinoleate.

3. Composition for neutralizing unpleasant odours in the environment in accordance with claim 1 **characterized in that** said compounds capable of lowering the vapour pressure of the gaseous molecules of the unpleasant odour are saturated aliphatic esters (C10-C20) or methylic esters of resinic acids.

4. Composition for neutralizing unpleasant odours in the environment in accordance with claim 3 **characterized in that** said saturated aliphatic ester (C10-C20) is isopropyl myristate and **in that** the resinic acid methylic ester is methyl dihydroabietate .

5. Composition for neutralizing unpleasant odours in the environment in accordance with claim 1 **characterized in that** said essential oils are obtained from the tea tree, Ceylon cinnamon leaves, citronella, artemisia, lemongrass, thyme, citrus fruits, anise, geranium, cedar, clove, rose, mint, lavender, eucalyptus, spearmint, sandalwood, pine, bergamot, lavandine or mixtures thereof; and the active ingredients are selected from amongst thymol, citronellal, citronellol, estragol, geranyl acetate, eucalyptol, geraniol, menthol, cedrol, anethol, limonene, acetol, carbacrol, eucalyptol, eugenol, terpineol, cynamaldehyde, cynamic acid, citral, nerol, dihydromircenol, rose oxide, limonene and mixtures thereof.

6. Composition for neutralizing unpleasant odours in the environment in accordance with claim 1 **characterized in that** the chemical, physical and biological neutralization systems are in the proportions of 3-15%, 3-25% and 10-40%, respectively.

7. Composition for neutralizing unpleasant odours in the environment in accordance with any of the preceding claims **characterized in that** it also comprises one or more aromatic compounds.

8. Use of a composition in accordance with any of claims 1-7 in order to eliminate unpleasant odours which comprises dispersing said composition in the environment.

## Patentansprüche

1. Zusammensetzung zum Neutralisieren unangenehmer Gerüche in der Umgebung, umfassend:
- wenigstens ein chemisches Neutralisationssystem auf der Basis eines Zinksalzes;
- wenigstens ein physikalisches Neutralisationssystem, das weiterhin eine Verbindung, die den Dampfdruck der den unangenehmen Geruch verursachenden gasförmigen Moleküle senken kann, und ein Coadjuvans, das die in der Luft verteilten festen Teilchen ausfällen kann, umfasst;
- und wenigstens ein biologisches Neutralisationssystem, das auf wenigstens einem etherischen Öl oder seinem aktiven Bestandteil beruht;
**dadurch gekennzeichnet, dass** das Coadjuvans auf einem Copolymer von Vinylpyrrolidon und Dimethylaminopropylmethacrylamid beruht.

2. Zusammensetzung zum Neutralisieren unangenehmer Gerüche in der Umgebung gemäß Anspruch 1, **dadurch gekennzeichnet, dass** es sich bei dem Zinksalz um Zinkricinoleat handelt.

3. Zusammensetzung zum Neutralisieren unangenehmer Gerüche in der Umgebung gemäß Anspruch 1, **dadurch gekennzeichnet, dass** die Verbindungen, die den Dampfdruck der den unangenehmen Geruch verursachenden gasförmigen Moleküle senken können, gesättigte aliphatische Ester (C10-C20) oder Methylester von Harzsäuren sind.

4. Zusammensetzung zum Neutralisieren unangenehmer Gerüche in der Umgebung gemäß Anspruch 3, **dadurch gekennzeichnet, dass** es sich bei dem gesättigten aliphatischen Ester (C10-C20) um Isopropylmyristat und bei dem Harzsäuremethylester um Methyldihydroabietat handelt.

5. Zusammensetzung zum Neutralisieren unangenehmer Gerüche in der Umgebung gemäß Anspruch 1, **dadurch gekennzeichnet, dass** die etherischen Öle von Teebaum, Ceylon-Zimtblättern, Citronella, Artemisia, Zitronengras, Thymian, Zitrusfrüchten, Anis, Geranium, Zeder, Gewürznelke, Rose, Minze, Lavendel, Eukalyptus, Speerminze, Sandelholz, Kiefer, Bergamotte, Lavandin oder Gemischen davon erhalten wurden und die aktiven Bestandteile aus Thymol, Citronellal, Citronellol, Estragol, Geranylacetat, Eucalyptol, Geraniol, Menthol, Cedrol, Anethol, Limonen, Acetol, Carbacrol, Eucalyptol, Eugenol, Terpineol, Zimtaldehyd, Zimtsäure, Citral, Nerol, Dihydromircenol, Rosenoxid, Limonen und Gemischen davon ausgewählt sind.

6. Zusammensetzung zum Neutralisieren unangenehmer Gerüche in der Umgebung gemäß Anspruch 1, **dadurch gekennzeichnet, dass** das chemische, das physikalische und das biologische Neutralisationssystem in den Anteilen 3-15%, 3-25% bzw. 10-40% vorhanden sind.

7. Zusammensetzung zum Neutralisieren unangenehmer Gerüche in der Umgebung gemäß einem der vorstehenden Ansprüche, **dadurch gekennzeichnet, dass** sie auch eine oder mehrere aromatische Verbindungen umfasst.

8. Verwendung einer Zusammensetzung gemäß einem der Ansprüche 1 bis 7 zur Beseitigung unangenehmer Gerüche, umfassend die Verteilung der Zusammensetzung in der Umgebung.

## Revendications

1. Composition pour neutraliser des odeurs désagréables dans l'environnement qui comprend
- au moins un système de neutralisation chimique basé sur un sel de zinc ;
- au moins un système de neutralisation physique comprenant en outre un composé capable d'abaisser la pression de vapeur des molécules gazeuses de l'odeur désagréable et un co-adjuvant capable de précipiter les particules solides en suspension dans l'air ;
- et au moins un système de neutralisation biologique basé sur au moins une huile essentielle ou son ingrédient actif,
**caractérisée en ce que** le co-adjuvant est basé sur un copolymère de vinylpyrrolidone et de diméthyl aminopropyl méthacrylamide.

2. Composition pour neutraliser des odeurs désagréables dans l'environnement selon la revendication 1, **caractérisée en ce que** ledit sel de zinc est le ricinoléate de zinc.

3. Composition pour neutraliser des odeurs désagréables dans l'environnement selon la revendication 1, **caractérisée en ce que** lesdits composés capables d'abaisser la pression de vapeur des molécules gazeuses de l'odeur désagréable sont des esters aliphatiques saturés (C₁₀ à C₂₀) ou des esters méthyliques d'acides résiniques.

4. Composition pour neutraliser des odeurs désagréables dans l'environnement selon la revendication 3, **caractérisée en ce que** ledit ester aliphatique saturé (C₁₀ à C₂₀) est le myristate d'isopropyle et **en ce que** l'ester méthylique d'acide résinique est le dihydroabiétate de méthyle.

5. Composition pour neutraliser des odeurs désagréables dans l'environnement selon la revendication 1, **caractérisée en ce que** lesdites huiles essentielles sont obtenues à partir du mélaleuca, des feuilles de cannelier, de la citronnelle, de l'armoise, du lemongrass, du thym, d'agrumes, de l'anis, du géranium, du cèdre, du clou de girofle, de la rose, de la menthe, de la lavande, de l'eucalyptus, de la menthe verte, du bois de santal, du pin, de la bergamote, du lavandin ou des mélanges de ceux-ci ; et les ingrédients actifs sont sélectionnés entre autres parmi le thymol, le citronellal, le citronellol, l'estragol, l'acétate de géranyle, l'eucalyptol, le géraniol, le menthol, le cédrol, l'anéthol, le limonène, l'acétol, le carbacrol, l'eucalyptol, l'eugénol, le terpinéol, le cynamaldéhyde, l'acide cynamique, le citral, le nérol, le dihydromircénol, l'oxyde de rose, le limonène et les mélanges de ceux-ci.

6. Composition pour neutraliser des odeurs désagréables dans l'environnement selon la revendication 1, **caractérisée en ce que** les systèmes de neutralisation chimiques, physiques et biologiques sont dans des proportions de 3 à 15 %, 3 à 25 % et 10 à 40 %, respectivement.

7. Composition pour neutraliser des odeurs désagréables dans l'environnement selon l'une quelconque des revendications précédentes, **caractérisée en ce qu'**elle comprend également un ou plusieurs composés aromatiques.

8. Utilisation d'une composition selon l'une quelconque des revendications 1 à 7, afin d'éliminer des odeurs désagréables qui comprend la dispersion de ladite composition dans l'environnement.
